# FASCICULE DE BREVET EUROPEEN

(11) **EP 2 127 323 B1**
(45) Date de publication et mention de la délivrance du brevet: **16.03.2016**
(21) Numéro de dépôt: 07857770.7
(22) Date de dépôt: 18.12.2007
(51) Int. Cl.: H04L 29/08, A61B 6/00, H04N 5/232, H04N 5/32

(54) **SYSTEME RADIOLOGIQUE NUMERIQUE ET PROCEDE DE MISE EN UVRE DU SYSTEME RADIOLOGIQUE**
DIGITALES RADIOLOGIESYSTEM UND VERFAHREN ZUR IMPLEMENTIERUNG DES RADIOLOGIESYSTEMS
DIGITAL RADIOLOGY SYSTEM AND METHOD FOR IMPLEMENTING THE RADIOLOGY SYSTEM

(30) Priorité: 21.12.2006 FR 0611211
(43) Date de publication de la demande: 02.12.2009
(73) Titulaire: Trixell S.A.S., 38430 Moirans (FR)
(72) Inventeur: CHASTEL, Olivier, F-38500 Voiron (FR)
(74) Mandataire: Collet, Alain
(86) Numéro de dépôt international: PCT/EP2007/064146
(87) Numéro de publication internationale: WO 2008/080837

(56) Documents cités:
- US-A- 5 773 832
- US-A- 5 828 726
- US-A- 5 844 961
- US-A1- 2004 079 908
- US-A1- 2006 097 177
- US-A1- 2006 242 094
- US-B1- 6 344 652

## Description

L'invention concerne un système radiologique numérique et un procédé de mise en oeuvre du système radiologique. Un système radiologique numérique comporte essentiellement plusieurs éléments tels qu'une source de rayonnement ionisant, comme par exemple un tube à rayons X, permettant de générer un rayonnement X, un détecteur de rayons X permettant de fournir une image fonction du rayonnement X reçu et une station de base comprenant un système de traitement de l'information permettant de synchroniser le tube à rayon X et le détecteur et permettant aussi de réaliser des traitements d'images comme de présenter à l'opérateur l'image corrigée de tous les défauts inhérents au détecteur et améliorée (par exemple par des traitements de rehaussement de contour). Un objet dont on veut obtenir l'image X est placé entre la source et le détecteur. Un tel système peut être utilisé dans de nombreuses applications telles que par exemple la radiologie médicale et le contrôle non destructif.

Par le passé, les systèmes radiologiques étaient volumineux et peu mobiles. Il était nécessaire de positionner l'objet par rapport au système pour obtenir l'image désirée. Avec l'apparition de détecteurs à l'état solide tels que par exemple décrit dans la demande de brevet français FR 2 605 166, le détecteur est devenu moins volumineux et il a été possible de déplacer le détecteur par rapport à un objet restant fixe. Pour la radiologie médicale on a réalisé des détecteurs numériques sous forme de cassettes mobiles qu'il devient possible de placer à proximité immédiate d'un patient dont on veut réaliser une image, lorsque l'état de santé du patient empêche son déplacement vers une salle réservée à la radiologie.

Pour assurer une grande disponibilité du système, la cassette est reliée à la station de base par une liaison filaire. Mais cette liaison limite la mobilité de la cassette. On pourrait imaginer de remplacer la liaison filaire par une liaison sans fil. Ceci est devenu possible avec l'apparition de technologies sans fils récentes et de composants électroniques à très faible consommation. Mais dans les systèmes radiologiques, des liaisons sans fil n'ont jamais été mises en oeuvre à cause des risques de perturbations de la liaison par exemple électromagnétiques réduisant la disponibilité du système. Par ailleurs, en cas de décharge complète de la batterie incluse dans la cassette, le système radiologique reste indisponible jusqu'à la recharge complète de la batterie (environ 2 heures). On a également mis en oeuvre des cassettes autonomes permettant l'acquisition et l'enregistrement de plusieurs images radiologiques. Ces cassettes sont périodiquement raccordées à la station de base par une liaison filaire pour assurer le déchargement des images. Cette opération de raccordement intervient après la prise d'image et ne permet donc pas l'utilisation instantanée des images.

Le document US 2006/097177 décrit un système de radiologie connecté au réseau numérique.

L'invention vise à pallier les problèmes cités plus haut en proposant un système radiologique mettant en oeuvre une liaison sans fil et une liaison filaire pour raccorder la cassette à la station de base.

A cet effet, l'invention a pour objet un système radiologique numérique comportant une cassette mobile et une station de base fixe, la cassette comprenant des moyens d'acquisition d'une image fonction d'un rayonnement X auquel la cassette est exposée, le système comportant en outre des moyens de communication entre la cassette et la station de base permettant de transférer des données telles que l'image entre la cassette et la station de base, caractérisé en ce que les moyens de communication comprennent une liaison filaire amovible et une liaison sans fil susceptibles toutes deux de transférer les données et en ce que le système comporte des moyens pour désactiver la liaison sans fil dès que la liaison filaire est établie.

L'invention a également pour objet un procédé de mise en oeuvre d'un système radiologique numérique comportant une cassette mobile et une station de base fixe, la cassette comprenant des moyens d'acquisition d'une image fonction d'un rayonnement X auquel la cassette est exposée, le système comportant en outre des moyens de communication entre la cassette et la station de base permettant de transférer des données telles que l'image entre la cassette et la station de base, les moyens de communication comprenant une liaison filaire amovible et une liaison sans fil susceptibles toutes deux de transférer les données, caractérisé en ce que le procédé consiste à établir un échange de données sur la liaison sans fil et à basculer l'échange vers la liaison filaire dès lors que celle ci est établie.

Avantageusement l'invention permet l'appariement de la station de base avec une cassette (par exemple en cas d'un remplacement dans le cadre du service après vente) dans un environnement où plusieurs systèmes radiologiques sans fils cohabitent.

L'invention sera mieux comprise et d'autres avantages apparaîtront à la lecture de la description détaillée d'un mode de réalisation donné à titre d'exemple, description illustrée par le dessin joint dans lequel :
La figure 1 représente schématiquement un système radiologique, comprenant une station de base et une cassette mobile, conforme à l'invention ;
la figure 2 représente plus en détail différents éléments de l'alimentation de la cassette ;
la figure 3 représente plus en détail un exemple de moyens de communication entre la cassette et la station de base ;
la figure 4 représente l'implantation de trois systèmes radiologiques chacun dans une salle afin de mieux comprendre un procédé d'appariement d'une station de base avec une cassette sans risque de confusion avec les cassettes des systèmes radiologiques voisins ;
la figure 5 représente sous forme d'organigramme l'appariement de la station de base et de la cassette.

Par souci de clarté, les mêmes éléments porteront les mêmes repères dans les différentes figures.

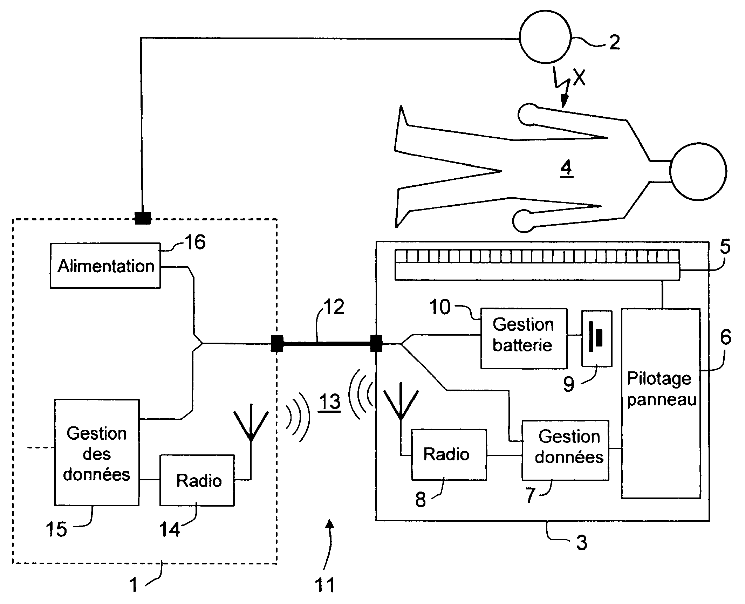

La figure 1 représente un système radiologique destiné à une utilisation médicale. Le système comporte une station de base fixe 1 un générateur de rayonnement X 2 et un détecteur de rayonnement sous forme d'une cassette mobile 3. La cassette permet d'obtenir une image d'un patient 4 traversé par le rayonnement X. La cassette 3 comporte un détecteur numérique réalisé sous forme d'un panneau plat 5 relié à un module de pilotage 6 permettant de lire l'image obtenue par le panneau plat 5 et de la numériser au travers d'un convertisseur analogique numérique. La cassette mobile 3 comporte également un module de gestion de données 7, un module radio 8, une batterie 9 et un module de gestion de la batterie 10.

La station de base comporte un module radio 14, un module de gestion de données 15 et une alimentation 16.

Des moyens de communication 11 entre la cassette 3 et la station de base 1 permettent de transférer des données telles que l'image entre la cassette 3 et la station de base 1. Les données peuvent circuler soit de la station de base 1 vers la cassette 3, soit de la cassette 3 vers la station de base 1. Vers la cassette 3, il s'agit par exemple d'informations de commande du panneau plat 5 et vers la station de base 1, les données comportent par exemple des images réalisées par le panneau plat 5.

Les moyens de communication comprennent une liaison filaire amovible 12 et une liaison sans fil 13. Les deux liaisons 12 et 13 sont susceptibles toutes deux de transférer les données. Les deux modules radio 8 et 14 permettent d'échanger les données entre la station de base 1 et la cassette 3. Le module de gestion de données 7 de la cassette 3 permet d'aiguiller les données reçues ou en provenance du module de pilotage 6 vers l'une des liaisons 12 ou 13. De même, dans la station de base 1, le module de gestion de données 15 permet d'aiguiller les données reçues ou en provenance d'une des liaisons 12 ou 13. L'alimentation 16 fournit l'énergie électrique nécessaire au fonctionnement des différents modules de la station de base 1 ainsi que de la cassette 3.

L'alimentation de la cassette 3 se fait par l'intermédiaire de la liaison filaire 12 ou de la batterie 9. Avantageusement, le système comporte des moyens pour recharger la batterie 9. Plus précisément, le module de gestion de la batterie 10 mesure la charge de la batterie 9 et provoque sa recharge en cas de besoin.

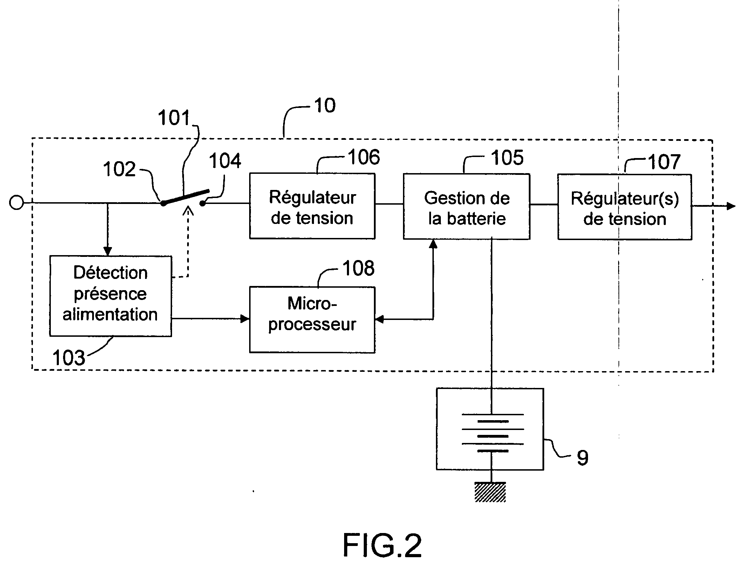

La figure 2 permet de mieux comprendre le fonctionnement du module de gestion de la batterie 10 et de la batterie 9. Le module de gestion de la batterie 10 comprend un interrupteur électronique 101 dont une première borne 102 permet de recevoir de l'énergie électrique de la station de base 1 par l'intermédiaire de la liaison filaire lorsque celle ci est établie. Un module de détection de présence de l'alimentation 103 ouvre l'interrupteur 101 lorsque la liaison filaire est interrompue. Le module 103 permet d'assurer la sécurité des utilisateurs de la cassette 3 et du patient 4 lorsqu'un connecteur de la cassette 3 recevant la liaison filaire 12 n'est pas raccordé. En effet, dans le milieu médical les courants maximum admissibles à travers un corps humain sont très faibles, de l'ordre de la dizaine de micro-ampère. Pour supprimer le risque d'électrisation on déconnecte les broches du connecteur du module de gestion de la batterie 10, au moyen de l'interrupteur 101. Une seconde borne 104 de l'interrupteur 101 est raccordée au coeur 105 du module 10 par l'intermédiaire d'un régulateur de tension 106 permettant de pallier d'éventuelles fluctuations de la tension de l'alimentation fournie par la liaison filaire 12. Ces fluctuations sont par exemple dues à la longueur de câble de la liaison filaire 12. Le coeur 105 contrôle notamment le courant de charge de la batterie 9. Un second régulateur de tension 107 fournit la puissance électrique aux autres composants de la cassette 3 tel que le panneau plat 5. Le module de gestion de la batterie 10 peut éventuellement comporter plusieurs seconds régulateurs 107 permettant d'alimenter divers composants de la cassette. Un microprocesseur 108 peut assurer la supervision de l'ensemble du module de gestion de la batterie 10. Le microprocesseur 108 peut également informer la station de base 1 du niveau de charge de la batterie 9 et de sa température.

Selon l'invention, le système radiologique comporte des moyens pour désactiver la liaison sans fil 13 dès que la liaison filaire 12 est raccordée. Le procédé de l'invention consiste à établir en priorité un échange de données sur la liaison sans fil 13 et à basculer l'échange vers la liaison filaire 12 lorsque celle ci est raccordée. La détection de raccordement de la liaison filaire 12 est par exemple réalisé par exemple au moyen du module de détection de présence de l'alimentation 103. L'alimentation électrique de la cassette 3 intervient dès que la liaison filaire 12 est établie.

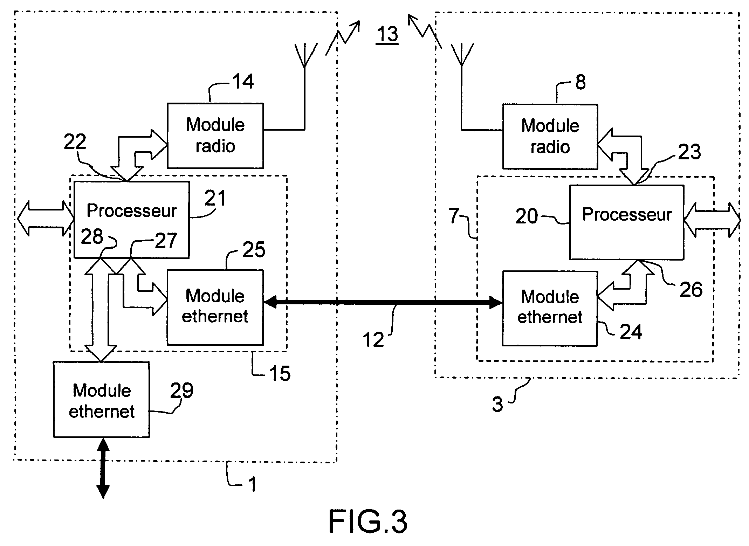

La figure 3 représente un exemple de réalisation des moyens de communication reliant la station de base 1 et la cassette 3. Chaque module de gestion des données 7 et 15 comporte un processeur, respectivement 20 et 21 avantageusement identiques. On peut par exemple mettre en oeuvre un processeur à jeu d'instruction réduit, bien connu dans la littérature anglo-saxonne sous le nom de processeur RISC. Ce type de processeur est bien optimisé pour des systèmes fonctionnant en temps réel. Chaque processeur comporte plusieurs ports permettant d'émettre et/ou de recevoir des flux de données. Un port 22 du processeur 21 est relié au module radio 14 et un port 23 du processeur 20 est relié au module radio 8. Les modules 8 et 14 adaptent le flux -de données émis et/ou reçu selon un protocole de transmission sans fil comme par exemple celui défini par la norme IEEE 802.11 (Institute of Electrical and Electronics Engineers, Inc.) bien connu dans la littérature anglo-saxonne sous le nom de WI-FI pour Wireless Fidelity. D'autres protocoles sont bien entendu utilisables comme le protocole Bluetooth par exemple. Ce protocole est défini par des entreprises majeures dans le domaine des télécommunications, de l'informatique et des réseaux telles que Agere, Ericsson, IBM, Intel, Microsoft, Motorola, Nokia et Toshiba.

Chaque module de gestion des données 7 et 15 comporte un module d'interface réseau, respectivement 24 et 25. Le module 24 est relié à un port 26 du processeur 20 et le module 25 est relié à un port 27 du processeur 21. Entre les modules 24 et 25 est raccordé le câble de la liaison filaire 12. Les modules 24 et 25 adaptent le flux de données émis et/ou reçu selon un protocole de transmission filaire comme par exemple celui défini par la norme IEEE 802.3 bien connu sous le nom de protocole Ethernet. D'autres protocoles sont possibles tels que RS 232 (norme définie par l'Electronic Industries Association basée aux Etats Unis), ou USB (Universal Serial Bus, norme définie par une association de constructeurs informatiques : USB Implementers Forum, Inc basée aux Etats Unis). On peut également mettre en oeuvre un protocole spécifique du système radiologique dit protocole propriétaire. La liaison filaire 12 peut par exemple utiliser un câble électrique ou optique. Les processeurs 20 et 21 peuvent comporter d'autres ports pour des échanges de données à l'intérieur ou vers l'extérieur de la station de base 1 et de la cassette 3. A titre d'exemple on a représenté un port 28 du processeur 21 relié à un module d'interface réseau 29. Pour la station de base 1, le module 29 est par exemple utilisé pour transmettre l'image réalisée par la cassette 3 vers des moyens de stockage et d'affichage.

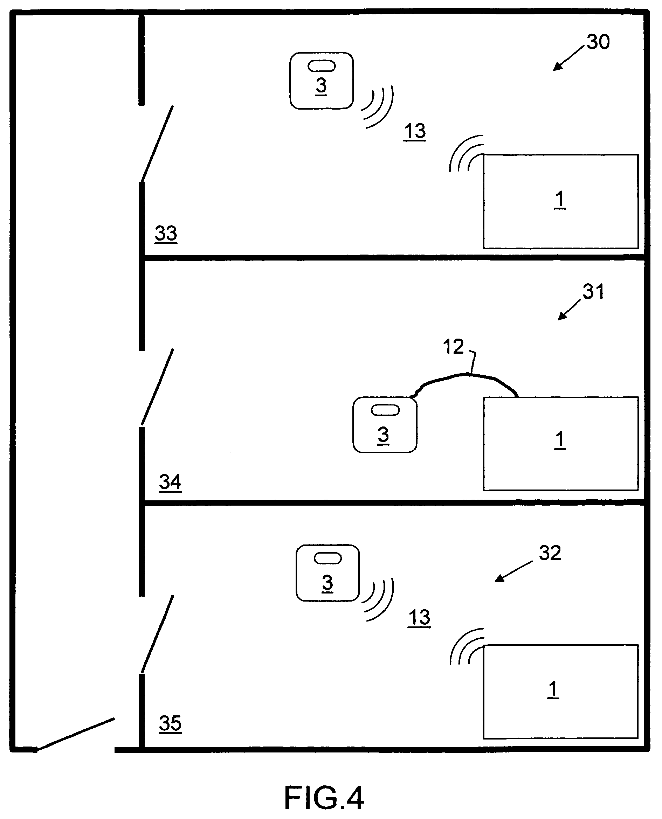

La figure 4 représente l'implantation de trois systèmes radiologiques 30, 31 et 32 chacun dans une salle, respectivement 33, 34 et 35. Pour l'échange de données, les systèmes radiologiques 30 et 32 utilisent leur liaison sans fil 13 et le système radiologique 31 utilise sa liaison filaire 12. L'échange de données entre une des stations de base 1 et la cassette 3 qui lui est associée doit être assuré sans risque de confusion avec une autre station de base 1 qui serait installée à proximité par exemple dans une salle voisine. Ce risque de confusion apparaît avec l'utilisation de la liaison sans fil 13. Dans le cas représenté sur la figure 4, une confusion risque d'apparaître entre les systèmes radiologiques utilisés dans les salles 33 et 35. Pour se faire, lors de l'échange de données au moins pour sur la liaison sans fil 13, la station de base 1 demande à la cassette 3 un identifiant et n'autorise la poursuite de l'échange de données qu'en cas de réponse correspondant à un identifiant mémorisé dans la station de base 1 de la part de la cassette 3. Ce contrôle est par exemple défini par la norme IEE 802.11. On peut imaginer d'apparier une station de base 1 et une cassette 3 en usine. Mais cette solution est très peu flexible. Cette solution empêche notamment le remplacement d'une cassette 3 sur site. Il est donc utile de créer une procédure d'appariement entre une station de base 1 et une cassette 3 sur site.

Avantageusement, le système radiologique comporte des moyens pour apparier la station de base 1 et la cassette 3 lorsque la liaison filaire 12 est établie. On entend par établissement de la liaison filaire le fait que le câble est raccordé et que des données transitent par la liaison filaire 12. Le procédé de l'invention consiste à apparier la station de base 1 et la cassette 3 au moyen de la liaison filaire 12. Plus précisément on autorise l'appariement que lorsque la liaison filaire 12 est établie et on l'interdit lorsque la liaison sans fil 13 est établie. En effet, il serait dangereux de réaliser un appariement entre une station de base 1 et une cassette 3 à l'aide de la liaison sans fil 13. Dans le cas représenté sur la figure 4 on risquerait d'associer les cassettes 3 des salles 33 et 35 à une seule station de base 1.

Avantageusement on apparie automatiquement une cassette 3 et une station de base 1 dès que la liaison filaire est établie. Cette procédure simplifie les manipulations du système radiologique. Néanmoins cet automatisme peut présenter certains risques d'appariements involontaires et on peut préférer que l'appariement n'intervienne qu'après une intervention volontaire d'un opérateur du système radiologique. Dans ce cas il est même possible d'apparier la cassette 3 et la station de base 1 qu'après une intervention protégée d'un opérateur du système radiologique. Autrement dit, il est nécessaire pour l'opérateur souhaitant faire l'appariement, de détenir une autorisation particulière pour réaliser cette opération. Cette opération peut se matérialiser par une clé par exemple physique ou encore sous forme d'un code secret que l'opérateur doit mettre en oeuvre pour accéder à la procédure d'appariement.

L'appariement consiste par exemple en un échange d'un identifiant et éventuellement d'une clé de chiffrement des données, le chiffrement étant mis en oeuvre dans le transfert des données sur la liaison sans fil 13 pour assurer la confidentialité des données échangées.

Les processeurs 20 et 21 de la casette 3 et de la station de base 1 fonctionnent chacun avec un logiciel de version définie. Les versions de ces logiciels peuvent évoluer indépendamment l'une de l'autre. Il est avantageux de vérifier la compatibilité des versions logiciel de la station de base 1 et de la casette 3 avant d'effectuer l'appariement proprement dit.

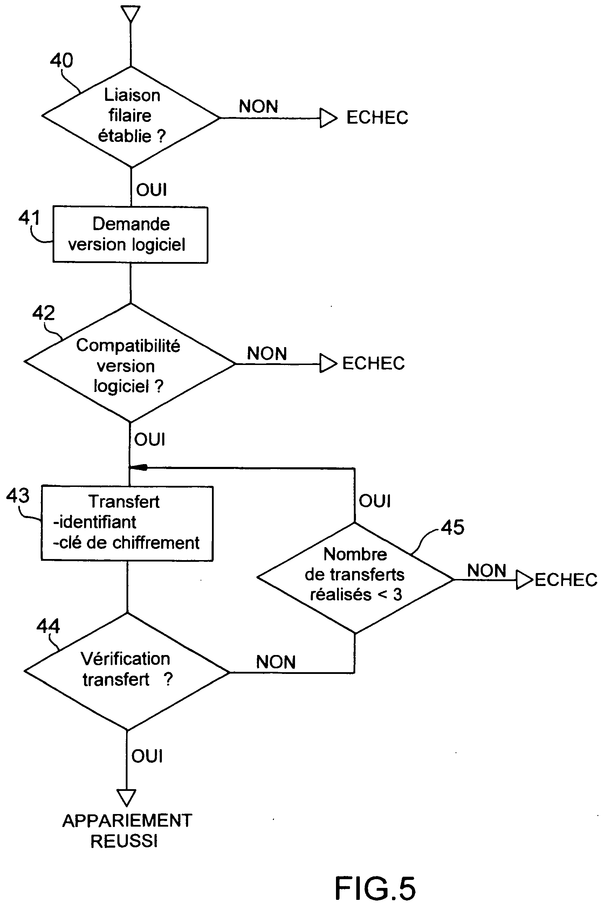

On peut résumer un procédé d'appariement par l'enchaînement avec succès des opérations illustrées sur la figure 5 :
- Vérification de l'établissement de la liaison filaire 12. Cette opération est représentée au cadre 40 sous forme d'un test. Si la liaison filaire 12 n'est pas établie, l'appariement est mis en échec et on en informe l'opérateur. Si au contraire, la liaison filaire 12 est établie, on poursuit par l'opération suivante :
- Vérification de la compatibilité des versions logiciel de la station de base 1 et de la cassette 3. Pour effectuer cette vérification, la station de base 1 interroge la cassette 3 sur sa version logiciel. Cette interrogation est représentée au cadre 41. La compatibilité est vérifiée par la station de base 1 par exemple à l'aide d'une base de donnée. Cette vérification de compatibilité est représentée au cadre 42. En cas d'incompatibilité, l'appariement est mis en échec et on en informe l'opérateur. Si au contraire, la compatibilité est avérée, on poursuit par l'opération suivante :
- Echange d'un identifiant et d'une clé de chiffrement des données, le chiffrement étant mis en oeuvre dans le transfert des données sur la liaison sans fil 13. Le transfert de l'identifiant et de la clé de chiffrement de la station de base 1 vers la cassette 3 est représenté au cadre 43 et une vérification du transfert est représentée au cadre 44. Cette vérification peut consister en une interrogation par la station de base 1 de la cassette 3 sur l'identifiant et la clé reçus que la station de base 1 compare avec ceux envoyés. En cas de différence entre les éléments envoyés et ceux reçus en retour on effectue, par exemple, jusqu'à deux nouvelles tentatives de transfert. Le comptage du nombres de tentatives est matérialisé au cadre 45. Au-delà de trois tentatives, si la vérification n'est pas correcte, l'appariement est mis en échec et on en informe l'opérateur.

## Revendications

1. Système radiologique numérique comportant une cassette (3) mobile et une station de base (1) fixe, la cassette (3) comprenant des moyens (5) d'acquisition d'une image fonction d'un rayonnement X auquel la cassette (3) est exposée, le système comportant en outre des moyens de communication (12, 13) entre la cassette (3) et la station de base (1) permettant de transférer des données telles que l'image entre la cassette (3) et la station de base (1), **caractérisé en ce que** les moyens de communication comprennent une liaison filaire (12) amovible et une liaison sans fil (13) susceptibles toutes deux de transférer les données et **en ce que** le système comporte des moyens pour désactiver la liaison sans fil (13) dès que la liaison filaire (12) est établie,
**en ce que** la cassette mobile (3) comporte un premier module radio (8) et un premier module de gestion de données (7) comprenant un premier processeur (20) et un premier module d'interface réseau (24) relié à un port (26) du premier processeur (20),
**en ce que** la station de base (1) comporte un second module radio (14) et un second module de gestion de données (15) comprenant un second processeur (21) et un second module d'interface réseau (25) relié à un port (27) du second processeur (21),
**en ce que** la liaison filaire (12) comprend un câble destiné à être raccordé entre les modules d'interface réseau (24, 25),
**en ce que** le premier module de gestion de données (7) de la cassette mobile (3) permet d'aiguiller les données reçues ou en provenance d'un module de pilotage (6) de la cassette mobile (3) vers l'une des liaisons (12, 13) filaire ou sans fil,
et **en ce que** le second module de gestion de données (15) de la station de base (1) permet d'aiguiller les données reçues ou en provenance d'une des liaisons (12, 13) filaire ou sans fil.

2. Système radiologique selon la revendication 1, **caractérisé en ce que** la cassette (3) comporte une batterie d'alimentation (9), **en ce que** le système comporte des moyens (10) pour recharger la batterie (9) comprenant un module de détection de présence de l'alimentation (103) et **en ce que** la détection de raccordement de la liaison filaire (12) est réalisée au moyen du module de détection de présence de l'alimentation (103).

3. Système radiologique selon l'une des revendications précédentes, **caractérisé en ce qu'**il comporte une batterie d'alimentation (9), **en ce que** le système comporte des moyens (10) pour recharger la batterie (9) comprenant un module de détection de présence de l'alimentation (103) et un interrupteur électronique (101) dont une première borne (102) permet de recevoir de l'énergie électrique de la station de base (1) par l'intermédiaire de la liaison filaire (12) lorsque celle ci est établie et **en ce que** le module de détection de présence de l'alimentation (103) ouvre l'interrupteur (101) lorsque la liaison filaire (12) est interrompue.

4. Système radiologique selon l'une des revendications précédentes, **caractérisé en ce qu'**il comporte des moyens (20, 21) pour autoriser l'appariement de la station de base (1) et de la cassette (3) lorsque la liaison filaire (12) est établie et pour interdire l'appariement lorsque la liaison sans fil (13) est établie.

5. Procédé de mise en oeuvre d'un système radiologique numérique comportant une cassette (3) mobile et une station de base (1) fixe, la cassette (3) comprenant des moyens (5) d'acquisition d'une image fonction d'un rayonnement X auquel la cassette (3) est exposée, le système comportant en outre des moyens de communication (12, 13) entre la cassette (3) et la station de base (1) permettant de transférer des données telles que l'image entre la cassette (3) et la station de base (1), les moyens de communication comprenant une liaison filaire (12) amovible et une liaison sans fil (13) susceptibles toutes deux de transférer les données, la cassette mobile (3) comportant un premier module radio (8) et un premier module de gestion de données (7) comprenant un premier processeur (20) et un premier module d'interface réseau (24) relié à un port (26) du processeur (20),
la station de base (1) comportant un second module radio (14) et un second module de gestion de données (15) comprenant un second processeur (21) et un second module d'interface réseau (25) relié à un port (27) du second processeur (21),
la liaison filaire (12) comprenant un câble destiné à être raccordé entre les modules d'interface réseau (24, 25), '
le premier module de gestion de données (7) de la cassette mobile (3) permettant d'aiguiller les données reçues ou en provenance d'un module de pilotage (6) de la cassette mobile (3)*vers l'une des liaisons (12, 13) filaire ou sans fil,
le second module de gestion de données (15) de la station de base (1) permettant d'aiguiller les données reçues ou en provenance d'une des liaisons (12, 13) filaire ou sans fil,
**caractérisé en ce que** le procédé consiste à établir en priorité un échange de données sur la liaison sans fil (13) et à basculer l'échange vers la liaison filaire (12) dès lors que celle ci est établie.

6. Procédé selon la revendication 5, **caractérisé en ce qu'**il consiste à alimenter la cassette (3) par la liaison filaire (12) dès que celle ci est établie.

7. Procédé selon l'une quelconques des revendications 5 ou 6, **caractérisé en ce qu'**il consiste à autoriser l'appariement de la station de base (1) et de la cassette (3) au moyen de la liaison filaire (12) et à interdire l'appariement au moyen de la liaison sans fil (13).

8. Procédé selon la revendication 7, **caractérisé en ce qu'**il consiste à apparier automatiquement la cassette (3) et la station de base (1) dès que la liaison filaire (12) est établie.

9. Procédé selon la revendication 7, **caractérisé en ce qu'**il consiste à apparier la cassette (3) et la station de base (1) après une intervention volontaire d'un opérateur du système radiologique.

10. Procédé selon la revendication 9, **caractérisé en ce qu'**il consiste à apparier la cassette (3) et la station de base (1) après une intervention protégée d'un opérateur du système radiologique.

11. Procédé selon l'une quelconques des revendications 7 à 10, **caractérisé en ce que** la station de base (1) et la cassette (3) fonctionne chacune avec un logiciel de version définie et **en ce que** l'appariement consiste à enchaîner avec succès les opérations suivantes :
• vérification de l'établissement de la liaison filaire (12) ;
• vérification de la compatibilité des versions logiciel de la station de base (1) et de la cassette (3) ;
• échange d'un identifiant et d'une clé de chiffrement des données, le chiffrement étant mis en oeuvre dans le transfert des données sur la liaison sans fil (13).

## Patentansprüche

1. Digitales Radiologiesystem, das eine bewegliche Kassette (3) und eine feste Basisstation (1) umfasst, wobei die Kassette (3) Mittel (5) zum Erfassen eines Bildes in Abhängigkeit von Röntgenstrahlung umfasst, der die Kassette (3) ausgesetzt ist, wobei das System ferner Mittel (12, 13) zum Kommunizieren zwischen der Kassette (3) und der Basisstation (1) umfasst, so dass Daten, wie zum Beispiel das Bild, zwischen der Kassette (3) und der Basisstation (1) übertragen werden können, **dadurch gekennzeichnet, dass** die Kommunikationsmittel eine abtrennbare verdrahtete Link (12) und eine drahtlose Link (13) umfassen, die beide die Daten übertragen können, und dadurch, dass das System Mittel zum Deaktivieren der drahtlosen Link (13) umfasst, sobald die verdrahtete Link (12) eingerichtet ist,
dadurch, dass die bewegliche Kassette (3) ein erstes Radiomodul (8) und ein erstes Datenmanagementmodul (7) umfasst, das einen ersten Prozessor (20) und ein erstes Netzschnittstellenmodul (24) umfasst, das mit einem Port (26) des ersten Prozessors (20) verbunden ist,
dadurch, dass die Basisstation (1) ein zweites Radiomodul (14) und ein zweites Datenmanagementmodul (15) umfasst, das einen zweiten Prozessor (21) und ein zweites Netzschnittstellenmodul (25) umfasst, das mit einem Port (27) des zweiten Prozessors (21) verbunden ist,
dadurch, dass die verdrahtete Link (12) ein Kabel zum Anschließen zwischen den Netzschnittstellenmodulen (24, 25) umfasst,
dadurch, dass das erste Datenmanagementmodul (7) der beweglichen Kassette (3) das Routen der von einem Steuermodul (6) der beweglichen Kassette (3) empfangenen oder davon kommenden Daten zu der verdrahteten oder der drahtlosen Link (12, 13) zulässt,
und dadurch, dass das zweite Datenmanagementmodul (15) der Basisstation (1) das Routen der von der verdrahteten oder der drahtlosen Link (12, 13) empfangenen oder davon kommenden Daten zulässt.

2. Radiologiesystem nach Anspruch 1, **dadurch gekennzeichnet, dass** die Kassette (3) eine Stromversorgungsbatterie (9) umfasst, dadurch, dass das System Mittel (10) zum Aufladen der Batterie (9) umfasst, umfassend ein Modul (103) zum Erkennen der Anwesenheit der Stromversorgung, und dadurch, dass die Erkennung des Anschlusses der verdrahteten Link (12) mittels des Moduls (103) zum Erkennen der Anwesenheit der Stromversorgung erfolgt.

3. Radiologiesystem nach einem der vorherigen Ansprüche, **dadurch gekennzeichnet, dass** es eine Stromversorgungsbatterie (9) umfasst, dadurch, dass das System Mittel (10) zum Aufladen der Batterie (9) umfasst, umfassend ein Modul (103) zum Erkennen der Anwesenheit der Stromversorgung und einen elektronischen Schalter (101), von dem ein erster Anschluss (102) den Empfang von elektrischer Energie von der Basisstation (1) über die verdrahtete Link (12) zulässt, wenn diese eingerichtet ist, und dadurch, dass das Modul (103) zum Erkennen der Anwesenheit der Stromversorgung den Schalter (101) öffnet, wenn die verdrahtete Link (12) unterbrochen ist.

4. Radiologiesystem nach einem der vorherigen Ansprüche, **dadurch gekennzeichnet, dass** es Mittel (20, 21) umfasst zum Zulassen des Paarens der Basisstation (1) und der Kassette (3), wenn die verdrahtete Link (12) eingerichtet ist, und zum Verhindern des Paarens, wenn die drahtlose Link (3) eingerichtet ist.

5. Verfahren zum Betreiben eines digitalen Radiologiesystems, das eine bewegliche Kassette (3) und eine feste Basistation (1) umfasst, wobei die Kassette (3) Mittel (5) zum Erfassen eines Bildes in Abhängigkeit von Röntgenstrahlung umfasst, der die Kassette (3) ausgesetzt ist, wobei das System ferner Mittel (12, 13) zum Kommunizieren zwischen der Kassette (3) und der Basisstation (1) umfasst, so dass Daten, wie zum Beispiel das Bild, zwischen der Kassette (3) und der Basisstation (1) übertragen werden können, wobei die Kommunikationsmittel eine abtrennbare verdrahtete Link (12) und eine drahtlose Link (13) umfassen, die beide die Daten übertragen können, wobei die bewegliche Kassette (3) ein erstes Radiomodul (8) und ein erstes Datenmanagementmodul (7) umfasst, das einen ersten Prozessor (20) und ein erstes Netzschnittstellenmodul (24) umfasst, das mit einem Port (26) des Prozessors (20) verbunden ist,
wobei die Basisstation (1) ein zweites Radiomodul (14) und ein zweites Datenmanagementmodul (15) umfasst, das einen zweiten Prozessor (21) und ein zweites Netzschnittstellenmodul (25) umfasst, das mit einem Port (27) des zweiten Prozessors (21) verbunden ist,
wobei die verdrahtete Link (12) ein Kabel zum Anschließen zwischen den Netzschnittstellenmodulen (24, 25) umfasst,
wobei das erste Datenmanagementmodul (7) der beweglichen Kassette (3) das Routen der von einem Steuermodul (6) der beweglichen Kassette (3) empfangenen oder davon kommenden Daten zu der verdrahteten oder der drahtlosen Link (12, 13) zulässt,
wobei das zweite Datenmanagementmodul (15) der Basisstation (1) das Routen der von der verdrahteten oder der drahtlosen Link (12, 13) empfangenen oder davon kommenden Daten zulässt,
**dadurch gekennzeichnet, dass** das Verfahren das Priorisieren der Einrichtung eines Austauschs von Daten über die drahtlose Link (13) und das Umschalten des Austauschs auf die verdrahtete Link (12) beinhaltet, sobald diese eingerichtet ist.

6. Verfahren nach Anspruch 5, **dadurch gekennzeichnet, dass** es das Versorgen der Kassette (3) über die verdrahtete Link (12) beinhaltet, sobald diese eingerichtet ist.

7. Verfahren nach Anspruch 5 oder 6, **dadurch gekennzeichnet, dass** es das Zulassen des Paarens der Basisstation (1) und der Kassette (3) mittels der verdrahteten Link (12) und das Verhindern des Paarens mittels der verdrahteten Link (13) beinhaltet.

8. Verfahren nach Anspruch 7, **dadurch gekennzeichnet, dass** es das automatische Paaren der Kassette (3) und der Basisstation (1) beinhaltet, sobald die verdrahtete Link (12) eingerichtet ist.

9. Verfahren nach Anspruch 7, **dadurch gekennzeichnet, dass** es das Paaren der Kassette (3) und der Basisstation (1) nach einer freiwilligen Intervention seitens eines Bedieners des Radiologiesystems beinhaltet.

10. Verfahren nach Anspruch 9, **dadurch gekennzeichnet, dass** es das Paaren der Kassette (3) und der Basisstation (1) nach einer geschützten Intervention seitens eines Bedieners des Radiologiesystems beinhaltet.

11. Verfahren nach einem der Ansprüche 7 bis 10, **dadurch gekennzeichnet, dass** die Basisstation (1) und die Kassette (3) jeweils mit einer definierten Software-Version arbeiten, und dadurch, dass das Paaren das erfolgreiche Verketten der folgenden Vorgänge beinhaltet:
• Überprüfen, dass die verdrahtete Link (12) eingerichtet ist;
• Überprüfen der Kompatibilität der Software-Versionen der Basisstation (1) und der Kassette (3);
• Austauschen einer Kennung und eines Datenverschlüsselungsschlüssels, wobei die Verschlüsselung bei der Übertragung von Daten über die drahtlose Link (13) erfolgt.

## Claims

1. A digital radiology system comprising a movable cassette (3) and a fixed base station (1), said cassette (3) comprising means (5) for acquiring an image as a function of X-rays to which said cassette (3) is exposed, said system further comprising means (12, 13) for communicating between said cassette (3) and said base station (1) allowing data, such as the image, to be transferred between said cassette (3) and said base station (1), **characterised in that** said communication means comprise a detachable wired link (12) and a wireless link (13) both capable of transferring the data, and **in that** said system comprises means for deactivating said wireless link (13) as soon as said wired link (12) is established,
**in that** said movable cassette (3) comprises a first radio module (8) and a first data management module (7) comprising a first processor (20) and a first network interface module (24) connected to a port (26) of said first processor (20),
**in that** said base station (1) comprises a second radio module (14) and a second data management module (15) comprising a second processor (21) and a second network interface module (25) connected to a port (27) of said second processor (21),
**in that** said wired link (12) comprises a cable intended to be connected between said network interface modules (24, 25),
**in that** said first data management module (7) of said movable cassette (3) allows the data received or originating from a control module (6) of said movable cassette (3) to be routed towards one of said wired or wireless links (12, 13),
and **in that** said second data management module (15) of said base station (1) allows the routing of the data received or originating from one of said wired or wireless links (12, 13).

2. The radiology system according to claim 1, **characterised in that** said cassette (3) comprises a power supply battery (9), **in that** said system comprises means (10) for recharging said battery (9) comprising a module (103) for detecting the presence of the power supply, and **in that** the detection of the connection of said wired link (12) is carried out by means of said module (103) for detecting the presence of the power supply.

3. The radiology system according to any one of the preceding claims, **characterised in that** it comprises a power supply battery (9), **in that** said system comprises means (10) for recharging said battery (9) comprising a module (103) for detecting the presence of the power supply and an electronic switch (101), a first terminal (102) of which allows electric power to be received from said base station (1) through said wired link (12) when said link is established, and **in that** said module (103) for detecting the presence of the power supply opens said switch (101) when said wired link (12) is interrupted.

4. The radiology system according to any one of the preceding claims, **characterised in that** it comprises means (20, 21) for authorising the matching of said base station (1) and said cassette (3) when said wired link (12) is established and for preventing the matching when said wireless link (3) is established.

5. A method for implementing a digital radiology system comprising a movable cassette (3) and a fixed base station (1), said cassette (3) comprising means (5) for acquiring an image as a function of X-rays to which said cassette (3) is exposed, said system further comprising means (12, 13) for communicating between said cassette (3) and said base station (1) allowing data, such as the image, to be transferred between said cassette (3) and said base station (1), said communication means comprising a detachable wired link (12) and a wireless link (13) both capable of transferring the data, said movable cassette (3) comprising a first radio module (8) and a first data management module (7) comprising a first processor (20) and a first network interface module (24) connected to a port (26) of said processor (20),
said base station (1) comprising a second radio module (14) and a second data management module (15) comprising a second processor (21) and a second network interface module (25) connected to a port (27) of said second processor (21),
said wired link (12) comprising a cable intended to be connected between said network interface modules (24, 25),
said first data management module (7) of said movable cassette (3) allowing the data received or originating from a control module (6) of said movable cassette (3) to be routed towards one of said wired or wireless links (12, 13),
said second data management module (15) of said base station (1) allowing the routing of the data received or originating from one of said wired or wireless links (12, 13),
**characterised in that** said method involves prioritising the establishing of an exchange of data over said wireless link (13) and switching the exchange to said wired link (12) as soon as said link is established.

6. The method according to claim 5, **characterised in that** it involves supplying said cassette (3) with power via said wired link (12) as soon as said link is established.

7. The method according to claim 5 or 6, **characterised in that** it involves authorising the matching of said base station (1) and said cassette (3) through said wired link (12) and preventing the matching through said wireless link (13).

8. The method according to 7, **characterised in that** it involves automatically matching said cassette (3) and said base station (1) as soon as said wired link (12) is established.

9. The method according to claim 7, **characterised in that** it involves matching said cassette (3) and said base station (1) after a voluntary intervention of an operator of said radiology system.

10. The method according to claim 9, **characterised in that** it involves matching said cassette (3) and said base station (1) after a protected intervention by an operator of said radiology system.

11. The method according to any one of claims 7 to 10, **characterised in that** said base station (1) and said cassette (3) each operate using a defined version of software and **in that** the matching involves successfully sequencing the following operations:
• verifying that said wired link (12) has been established;
• verifying the compatibility of the software versions of said base station (1) and of said cassette (3);
• exchanging an identifier and a data encryption key, said encryption being implemented in the transfer of data over said wireless link (13).
